# EUROPÄISCHE PATENTSCHRIFT

(11) **EP 0 765 393 B1**
(45) Veröffentlichungstag und Bekanntmachung des Hinweises auf die Patenterteilung: **03.12.2003**
(21) Anmeldenummer: 95923329.7
(22) Anmeldetag: 14.06.1995
(51) Int. Cl.: C12N 15/29, C12N 15/82, C07K 14/415, C12N 1/21, C12N 1/19, A01H 5/00

(54) **DNA-MOLEKÜLE, DIE FÜR EINEN PLASTIDÄREN 2-OXOGLUTARAT/MALAT-TRANSLOKATOR CODIEREN**
DNA MOLECULES WHICH CODE FOR A PLASTID 2-OXOGLUTARATE/MALATE TRANSLOCATOR
MOLECULES D'ADN CODANT POUR UN TRANSLOCATEUR PLASTIDIAL DE 2-OXOGLUTARATE/MALATE

(30) Priorität: 15.06.1994 DE 4420782
(43) Veröffentlichungstag der Anmeldung: 02.04.1997
(73) Patentinhaber: BASF AKTIENGESELLSCHAFT, 67056 Ludwigshafen (DE)
(72) Erfinder: Fischer, Karsten, Dr., 50354 Hürth-Efferen (DE); Flügge, Ulf-Ingo, Prof. Dr., 50997 Köln (DE); Weber, Andreas, Dr., 50937 Köln (DE)
(74) Vertreter: VOSSIUS & PARTNER
(86) Internationale Anmeldenummer: EP9502319
(87) Internationale Veröffentlichungsnummer: WO95034654

(56) Entgegenhaltungen:
- EP-A- 0 255 340
- WO-A-93/10251
- WO-A-94/01559
- WO-A-94/10320
- BIOCHIM BIOPHYS ACTA 1147 (1). 1993. 13-18, MENZLAFF E., ET AL. 'PURIFICATION AND FUNCTIONAL RECONSTITUTION OF THE 2-OXOGLUTARATE/MALATE TRANSLOCATOR FROM SPINACH CHLOROPLASTS.'
- BIOCHEMISTRY 34 (8). 1995. 2621-2627., WEBER A 'The 2-oxoglutarate-malate translocator of chloroplast envelope membranes: Molecular cloning of a transporter containing a 12-helix motif and expression of the functional protein in yeast cells.'
- EMBL SEQUENCE DATABASE, REL.42,13-DEC-1994 ACC. NO. U13238,SPINACIA OLERACEA POLKA CHLOROPLAST ENVELOPE MEMBRANE 2-OXOGLUTARATE/MALATE TRANSLOCATOR
- BIOCHEMISTRY, Bd. 29, 1990 Seiten 11033-11040, RUNSWICK, M.J., ET AL. 'SEQUENCE OF THE BOVINE 2-OXOGLUTARATE/MALATE CARRIER PROTEIN: STRUCTURAL RELATIONSHIP TO OTHER MITOCHONDRIAL TRANSPORT PROTEINS'
- THE PLANT JOURNAL, Bd. 5, Nr. 2, Februar 1994 Seiten 215-226, FISCHER, K., ET AL. 'CLONING AND IN VIVO EXPRESSION OF FUNCTIONAL TRIOSE PHOSPHATE/PHOSPHATE TRANSLOCATORS FROM C3- AND C4-PLANTS: EVIDENCE FOR THE PUTATIVE PARTICIPATION OF SPECIFIC AMINO ACID RESIDUES IN THE RECOGNITION OF PHOSPHOENOLPYRUVATE'
- PLANT PHSYIOLOGY, Bd. 96, 1991 Seiten 1003-1007, GENCHI, G., ET AL. 'PARTIAL PURIFICATION AND RECONSTITUTION OF THE ALPHA-KETOGLUTARATE CARRIER FROM CORN (ZEA MAYS L.) MITOCHONDRIA'
- PROCEEDINGS OF THE NATIONAL ACADEMY OF SCIENCES OF USA, Bd. 90, Juli 1993 WASHINGTON US, Seiten 6160-6164, RIESMEIER, J.W., ET AL. 'ANTISENSE REPRESSION OF THE CHLOROPLAST TRIOSE PHOSPHATE TRANSLOCATOR AFFECTS CARBON PARTITIONING IN TRANSGENIC POTATO PLANTS' in der Anmeldung erwähnt

## Beschreibung

Die vorliegende Erfindung betrifft DNA-Moleküle, insbesondere aus *Spinacia oleracea*, die den codierenden Bereich eines 2-Oxoglutarat/Malat-Transporters enthalten und deren Einführung in ein pflanzliches Genom die Bildung und Weiterleitung von Kohlenstoffgrundgerüsten für die Fixierung von Stickstoff und die Weiterleitung des assimilierten Stickstoffs in transgenen Pflanzen verändert. Ferner betrifft die Erfindung Plasmide, Hefen und Bakterien enthaltend diese DNA-Moleküle, sowie transgene Pflanzen, bei denen durch Einführung der DNA-Moleküle Veränderungen der Aktivität des 2-Oxoglutarat/Malat-Transporters und somit Änderungen im Stickstoff- und Kohlenstoffstoffwechsel hervorgerufen werden. Weiterhin betrifft die Erfindung transgene Pflanzen, die durch die Veränderung der Aktivität des 2-Oxoglutarat/Malat-Transporters in ihrer Fähigkeit zur Photorespiration beeinflußt sind. Ebenso betrifft die Erfindung die Benutzung der beschriebenen DNA-Moleküle, die für einen 2-Oxoglutarat/Malat-Translokator codieren, zur Identifizierung verwandter Translokatoren aus *Spinacia oleracea* und anderen Pflanzen durch Hybridisierung mit niedriger Stringenz oder durch PCR-Techniken, sowie die Nutzung des 2-Oxoglutarat/Malat-Translokators als Ziel ("Target") für Herbizide.

Lediglich Pflanzen, Bakterien und Hefen sind zur Überführung von anorganischem Stickstoff (Nitratstickstoff) in organisch gebundenen Stickstoff in großem Maßstab (in der Regel in Form von Aminosäuren) durch reduktive Aminierung von organischen Kohlenstoffverbindungen befähigt. Der Rest der belebten Welt, insbesondere Nutztiere und Menschen, ist auf Pflanzen als primäre Lieferanten organischer Stickstoffverbindungen angewiesen. Die Assimilation von anorganischem Stickstoff in Pflanzen durch Bindung an organischen Kohlenstoff hängt von der Verfügbarkeit von Stickstoff, Kohlenstoff-Grundgerüsten und Energie ab.
Die Stickstoffversorgung der Pflanze ist durch Düngung zu beeinflussen. Die Energie für die Stickstoffassimilation stammt aus der Lichtreaktion der Photosynthese bzw. in Wurzeln und anderen nicht-grünen Geweben aus der Dissimilation und ist unter normalen Feldbedingungen kein begrenzender Faktor.

2-Oxoglutarat (α-Ketoglutarat) ist nach heutigem Kenntnisstand der primäre Akzeptor reduzierten Stickstoffs in der Glutaminsynthase/Glutamin-2-Oxoglutarat-Aminotransferase (GOGAT)-Reaktion. In dieser Reaktionsfolge wird zunächst durch die Glutaminsynthase reduzierter Stickstoff (Ammoniumstickstoff) unter Energieverbrauch auf Glutamat übertragen. Es entsteht Glutamin. In einer Folgereaktion katalysiert nun die Glutamat-Oxoglutarat-Aminotransferase (GOGAT; Glutamatsynthase) unter Verbrauch von Reduktionsäquivalenten die Übertragung einer Aminogruppe von Glutamin auf 2-Oxoglutarat (Transaminierung). Es entstehen zwei Moleküle Glutamat.

Die gesamte Reaktionsfolge der Glutaminsynthase/GOGAT-Reaktion ist im Stroma der Plastiden der Pflanzen lokalisiert. Diese Organelle sind von zwei Lipiddoppelschichtmembranen umschlossen, wobei die äußere Molekularsiebcharakter hat und Verbindungen bis zu einer Größe von ca. 10 kDa frei passieren läßt (Flügge und Benz, 1984, FEBS Lett. 169: 85-89). Die innere Membran ist permeabel für einige kleinere Verbindungen wie Wasser, Kohlendioxid, Sauerstoff und Nitrit, nicht jedoch für größere geladene Moleküle wie zum Beispiel 2-Oxoglutarat.
Die Schlüsselverbindung der Glutaminsynthase/GOGAT-Reaktion, das 2-Oxoglutarat, muß aus dem Cytosol der pflanzlichen Zelle durch einen spezifischen Translokator über die innere Chloroplastenhüllmembran in das Stroma des Plastiden bewegt werden. Der Transport von 2-Oxoglutarat in die Plastiden erfolgt im Gegentausch mit Malat aus den Plastiden durch den 2-Oxoglutarat/Malat-Translokator. Das bei diesem Vorgang in das Cytosol exportierte Malat wird von einem zweiten, in seiner Substratspezifität dem 2-Oxoglutarat/Malat-Translokator verwandten Translokator, dem Dicarboxylat-Translokator, im Gegentausch mit Glutamat zurücktransportiert. Im Ergebnis wird ohne einen Nettotransport von Malat, das über beide Translokatorsysteme zirkuliert ("Doppel-Translokator", Woo et al., 1987, Plant Physiol. 84: 624-632; Flügge et al., 1988, Planta 174: 534-541), 2-Oxoglutarat in den Chloroplasten importiert und das Endprodukt der Glutaminsynthase/GOGAT-Reaktion, Glutamat, exportiert. Glutamat dient in der Pflanze als bevorzugter Aminogruppen-Donator in einer ganzen Reihe von Transaminierungsreaktionen, zum Beispiel bei der Biosynthese der Aminosäuren Alanin oder Phenylalanin etc. Weiterhin ist Glutamat eine wichtige Transportform für organisch gebundenen Stickstoff innerhalb der Pflanze. Die meisten stickstoffhaltigen Verbindungen in der Pflanze wie Aminosäuren, Nucleinsäuren oder Alkaloide benötigen in ihrem Biosyntheseweg zunächst Glutamat als primären Aminogruppendonator.

Das für die Stickstoffassimilation benötigte 2-Oxoglutarat wird im wesentlichen durch Umformung von Citrat im Cytoplasma der Zellen synthetisiert.
In neueren Publikationen (Riesmeier et al., 1993 Proc. Natl. Acad. Sci. USA 90: 6160-6164; Heineke et al., 1994, Planta 193: 174 - 180) wurde gezeigt, daß die Effektivität der photosynthetischen Kohlenstoffreaktion u.a. wesentlich durch den Abtransport des entstandenen reduzierten, organisch gebundenen Kohlenstoffs (Triose-Phosphat), der durch einen in der inneren Chloroplastenhüllmembran lokalisierten Translokator katalysiert wird, limitiert wird. Dieses Translokatorprotein stellt demzufolge eine Engstelle im Kohlenstoffmetabolismus dar. Eine ähnliche Rolle kommt im Fall des Stickstoffmetabolismus dem plastidären 2-Oxoglutarat/Malat-Translokator zu.

Der plastidäre 2-Oxoglutarat/Malat-Translokator nimmt somit im Stickstoffmetabolismus der Pflanzen eine zentrale Stellung ein, da er für die Bereitstellung ausreichender Mengen des Substrates für die Stickstoffassimilation durch die Glutaminsynthase/GOGAT-Reaktion verantwortlich ist. Eine Manipulation der Aktivität dieses Translokators würde daher eventuell eine Beeinflussung der Effektivität der Stickstoffassimilation in Pflanzen ermöglichen

Da der überwiegende Teil der Menschen auf der Erde auf pflanzliche Ernährung angewiesen ist, mit der Konsequenz einer ständigen Unterversorgung an Eiweißen in diesen Bevölkerungsschichten, besteht ein dringender Bedarf an Pflanzen mit einem erhöhten Anteil an organischen Stickstoffverbindungen, insbesondere Proteinen und Aminosäuren. Ebenso wird in der Tierfütterung in den entwickelten Ländern zunehmend Tier- und Fischmehl dem Viehfutter beigemengt, um die Eiweißversorgung der Zuchttiere zu verbessern. Futterpflanzen mit höherem Proteingehalt wären hier sicher die bessere Alternative, insbesondere mit Blick auf die durch die Verfütterung von Tiermehlen entstehenden Probleme wie BSE etc.

Eine Beeinflussung der Aktivität des plastidären 2-Oxoglutarat/Malat-Translokators mittels gentechnologischer Methoden wäre möglich, falls DNA-Sequenzen, die für einen derartigen Translokator codieren zur Verfügung stünden. Dies ist bisher jedoch nicht der Fall. Die Bereitstellung von DNA-Sequenzen, die für einen 2-Oxoglutarat/Malat-Translokator codieren, würde es ferner ermöglichen, Substanzen zu identifizieren, die spezifisch diesen Translokator inhibieren und sich somit als Herbizide verwenden lassen.

Gegenwärtig sind Sequenzen von Translokator-Proteinen der beschriebenen Substratspezifität nur aus den Mitochondrien von Rinderherzen und aus menschlichen Mitochondrien bekannt (Runswick et al., 1990, Biochemistry 29: 11033-11040; Iacobazzi et al., 1992, DNA Seq. 3(2): 79-88). Diese Transporter spielen eine wichtige Rolle im mitochondrialen Dicarbonsäure-Stoffwechsel (u.a. Malat/Aspartat-Shuttle, Oxoglutarat/Isocitrat-Shuttle) und sind der Familie mitochondrialer Metabolittransporter zugehörig, die untereinander nahe verwandt sind. So sind die mitochondrialen Carrier (Phosphat/OH⁻, ADP/ATP, Oxoglutarat/Malat etc.) durch Sequenzverwandtschaft und durch das Vorkommen von internen Sequenzwiederholungen ("internal repeats") gekennzeichnet. Weiterhin konnte für die meisten mitochondrialen Carrier, wie auch für den 2-Oxoglutarat-Carrier gezeigt werden, daß diese ohne eine sie zu den Organellen dirigierende Präsequenz (Adressierungs-Sequenz, "Targeting"-Sequenz) in die Mitochondrien-Membran eingebaut werden (Runswick et al., 1990, Biochemistry 29: 11033-11040). Es ist also anzunehmen, daß die "Targeting"-Information im reifen Carrierprotein enthalten ist. Eine Überexpression eines mitochondrialen Dicarbonsäure-Transporters in Pflanzen würde somit nicht zu einer Erhöhung des Oxoglutarat-Transportes über die Plastidenhüllmembran führen, sondern nur zur Verstärkung des mitochondrialen Dicarbonsäure-Transportes, ein nicht erwünschter Effekt.

Im Gegensatz hierzu benötigen Proteine der inneren Plastidenhüllmembran eine Präsequenz, die spezifisch diese Proteine zu den Plastiden dirigiert (Übersichtsartikel hierzu: Keegstra et al., 1989, Annu. Rev. Plant Physiol. Plant Mol. Biol. 40: 471-501; Lubben et al., 1988, Photosynth. Res. 17: 173-194; Flügge, 1990, J. Cell Sci. 96: 351-354). Neben der für die "Plastiden-Adressierung" nötigen Präsequenz gibt es im reifen Teil (der Teil des Proteins, der nach der Abspaltung der Präsequenz durch eine spezifische Protease verbleibt) der Plastidenhüllmembran-Proteine noch weitere Informationen, die einen Transport der Hüllmembranproteine über die Hüllmembran in das Plastidenstroma oder die Thylakoidmembran verhindern (eigene, unveröffentlichte Beobachtungen; Li at al., 1992, J. Biol. Chem. 267: 18999-19004). Die genaue Lokalisation dieser Informationen im reifen Teil der bisher bekannten Proteine der inneren Hüllmembran (37 kDa-Protein: Dreses-Werringloer et al., 1991, Eur. J. Biochem. 195: 361-368; Triosephosphat/Phosphat-Translokator: Flügge et al., 1989, EMBO J. 8: 39-46; Willey et al., 1991, Planta 183: 451-461; Fischer et al., 1994, Plant Jour. 5(2): 215-226; Ca²⁺-ATPase: Huang et al., 1993, Proc. Natl. Acad. Sci. USA 90: 10066-10070) gelang bisher nicht. Eigene Untersuchungen zeigten am Beispiel eines mitochondrialen Carriers, des ADP/ATP-Transporters, daß dieses Protein nicht oder nur mit einer sehr geringen Effizienz zu den Chloroplasten dirigiert und dort in die Hüllmembran eingebaut werden kann. Selbst ein Hybridprotein, bestehend aus einer chloroplastidären Präsequenz (die Information für die Chloroplasten-Adressierung enthaltend) und diesem mitochondrialen Carrier, zeigte gegenüber dem authentischen Protein einen kaum erhöhten Einbau in die Chloroplasten-Hüllmembran (unveröffentlichte Beobachtungen). Da für eine korrekte Insertion die Protein/Lipid-Wechselwirkung von Bedeutung ist und sich die chloroplastidäre Hüllmembran in ihrer Lipidzusammensetzung grundlegend von der der Mitochondrien unterscheidet (Joyard et al., 1991, Eur. J. Biochem. 199: 489-509), ist es sehr unwahrscheinlich, daß eine, wenn auch geringfügige Insertion, eines mitochondrialen Proteins, dann auch funktionell ist d.h., daß ein Transporter aus anderen Organellen überhaupt in einer seiner Funktion entsprechenden Konformation und Orientierung in die Chloroplasten-Hüllmembran eingebaut werden kann.

Somit ist es nach gegenwärtigem Stand der Technik nicht möglich, mit Hilfe bekannter Plastiden-"Targeting"-Sequenzen z.B. mitochondriale oder prokaryontische Dicarbonsäure-Transporter funktionell in die innere Plastidenhüllmembran zu integrieren. Beim gegenwärtigen Stand der Technik ist es erfolgversprechender, die für die Konstruktion der oben geschilderten Pflanzen notwendige DNA-Sequenz durch Clonierung des authentischen plastidären 2-Oxoglutarat/Malat-Translokators zu erhalten.

Diese Clonierung kann jedoch nicht durch niedrig stringente Durchmusterung einer pflanzlichen cDNA-Bibliothek mit einer von den mitochondrialen Dicarbonsäure-Transportern abgeleiteten Sonde erfolgen, da nach bisherigem Kenntnisstand die chloroplastidären Translokatoren eine zu allen Translokatoren aus anderen Systemen (Bakterien, Mitochondrien) - selbst bei vergleichbarer Funktion des Proteins - völlig unterschiedliche Primärsequenz aufweisen. Es mußte daher zunächst der bei Membranproteinen außerordentlich schwierige Weg der biochemischen Charakterisierung, Reinigung und Isolation des 2-Oxoglutarat/Malat-Translokators beschritten werden. Es ist nun gelungen, das Translokator-Protein als Komponente der inneren Chloroplastenhüllmembran mit einem apparenten Molekulargewicht von 45 000 Dalton zu identifizieren (Menzlaff und Flügge, 1993, Biochim. Biophys. Acta 1147: 13-18). Die beschriebene Reinigungsprozedur ist jedoch nicht geeignet, für eine Proteinsequenzierung ausreichende Mengen Protein zu präparieren, zumal sich ergab, daß der N-Terminus des Proteins blockiert und somit einer N-terminalen Proteinsequenzierung durch automatisierten Edman-Abbau nicht zugänglich war. Daher war es bisher nicht möglich ausgehend von der Aminosäuresequenz DNA-Moleküle zu isolieren, die für einen plastidären 2-Oxoglutarat/Malat-Translokator codieren.

Der vorliegenden Erfindung liegt somit die Aufgabe zugrunde, Verfahren und DNA-Moleküle zur Verfügung zu stellen, mit deren Hilfe es möglich ist, Pflanzen dahingehend zu verändern, daß sie in der Lage sind, einen erhöhten Anteil an organischen Stickstoffverbindungen zu synthetisieren. Insbesondere liegt der Erfindung die Aufgabe zugrunde, DNA-Moleküle zur Verfügung zu stellen, die für einen plastidären 2-Oxoglutarat/Malat-Translokator codieren.

Diese Aufgabe wird durch die Bereitstellung der in den Patentansprüchen gekennzeichneten Ausführungsformen gelöst.

Somit betrifft die vorliegende Erfindung DNA-Moleküle, die für einen plastidären 2-Oxoglutarat/Malat-Translokator codieren, insbesondere DNA-Moleküle, die für ein Protein mit der unter Seq ID No. 1 angegebenen Aminosäuresequenz codieren, sowie DNA-Moleküle, die den unter Seq ID No. 1 angegebenen codierende Bereich umfassen. Ferner betrifft sie DNA-Moleküle, die mit den obengenannten erfindungsgemäßen DNA-Molekülen hybridisieren und DNA-Moleküle, deren Sequenz im Vergleich zu diesen aufgrund des genetischen Codes degeneriert ist, wobei diese Moleküle jeweils für ein Protein mit 2-Oxoglutarat/Malat-Translokatoraktivität codieren. Gegenstand der Erfindung sind ebenso DNA-Moleküle, die zu den obengenannten komplementär sind, sowie Fragmente und Derivate der obengenannten erfindungsgemäßen DNA-Moleküle, die beispielsweise durch Insertion, Substitution oder Deletion von diesen abgeleitet sein können, und die für einen 2-Oxoglutarat/Malat-Translokator codieren.

Unter dem Begriff Hybridisierung wird in diesem Zusammenhang eine Hybridisierung unter konventionellen Bedingungen wie z.B. in Sambrook et al. (1989, Molecular Cloning, A Laboratory Manual, 2. Aufl., Cold Spring Harbor Laboratory Press, Cold Spring Harbor, NY) beschrieben, verstanden, vorzugsweise eine Hybridisierung unter stringenten Bedingungen (ebenfalls beschrieben in Sambrook et al., s.o.).

Die erfindungsgemäßen DNA-Moleküle (oder Derivate oder Teile dieser Moleküle) können in Plasmide eingebracht werden, die eine Mutagenese oder eine Sequenzveränderung durch Rekombination von DNA-Sequenzen in prokaryontischen oder eukaryontischen Systemen erlauben. Dadurch kann die Spezifität des 2-Oxoglutarat/Malat-Translokators verändert werden. Durch eine Veränderung der Spezifität des Translokators in Richtung einer verbesserten Spezifität für Glutamat bei verringerter Spezifität für Malat könnte das oben beschriebene "Doppel-Translokator"-System mit dem Ziel weiter erhöhter Transportraten für 2-Oxoglutarat im direkten Austausch mit Glutamat verändert werden. Ebenso könnte eine Unempfindlichkeit gegenüber für den 2-Oxoglutarat/Malat-Translokator spezifischer Herbizide erreicht werden. Mit Hilfe von Standardverfahren (Sambrock et al., 1989, Molecular cloning: A laboratory manual, 2. Aufl., Cold Spring Harbor Laboratory Press, NY, USA) können Basenaustausche und/oder Basendeletionen vorgenommen und/oder synthetische oder natürliche Sequenzen hinzugefügt werden. Für die Verbindung der DNA-Fragmente untereinander können Adapter oder *linker* angesetzt werden. Ferner können Manipulationen, die passende Restriktionsschnittstellen bereitstellen oder die nicht benötigte DNA entfernen, eingesetzt werden. Dort wo Insertionen, Deletionen oder Substitutionen wie z.B. Transitionen oder Transversionen in Frage kommen, können *in vitro*-Mutagenese, "*primerrepair*", Restriktion oder Ligation verwendet werden. Als Analysemethode werden im allgemeinen eine Sequenzanalyse, eine Restriktionsanalyse und weitere biochemisch-molekularbiologische Methoden wie z.B. die Expression des modifizierten Proteins in Spalthefe und die Messung der modifizierten Transporteigenschaften in artifiziellen Liposomen (siehe Beispiel 4 und Loddenkötter et al., 1993, Proc. Natl. Acad. Sci. USA 90: 2155-2159;. sowie Fischer et al., 1994, Plant Journal 5: 215-226) oder die Messung der modifizierten Transporteigenschaften am in transgenen Pflanzen exprimierten Protein mit Hilfe einer kürzlich von den Erfindern zu diesem Zweck entwickelten Methode (Flügge und Weber, 1994, Planta, 194: 181-185).

Die erfindungsgemäßen DNA-Moleküle (oder Teile oder Derivate dieser Moleküle) können dazu genutzt werden, nach Standardverfahren aus dem Genom von Pflanzen ähnliche DNA-Moleküle zu isolieren, die ebenfalls für einen 2-Oxoglutarat/Malat-Translokator oder einen ähnlichen Dicarbonsäure-Translokator codieren. Als Methoden kommen dabei insbesondere in Betracht die Hybridisierungs-Durchmusterung von cDNA-Bibliotheken mit niedriger Stringenz unter Verwendung der erfindungsgemäßen DNA-Moleküle oder Teilen davon als Sonde oder die Erstellung von Sonden für stringente und niedrig stringente Durchmusterungs-Strategien durch Ableitung von degenerierten und/oder nicht degenerierten Primern aus den Sequenzen der erfindungsgemäßen DNA-Moleküle für PCR-Experimente mit DNA oder cDNA von Spinat oder anderen Pflanzen. Ferner können die erfindungsgemäßen DNA-Moleküle verwendet werden, um DNA-Moleküle zu identifizieren und zu isolieren, die den nahe verwandten Glutamat-Malat-Translokator (Dicarbonsäure-Translokator) der Plastidenhüllmembran codieren (subtraktive Durchmusterungsverfahren einer cDNA-Bibliothek aus Spinat oder anderen Pflanzen unter verschiedenen Stringenzbedingungen und mit verschiedenen Bereichen der erfindungsgemäßen DNA-Moleküle als Sonde).

In einer weiteren Ausführungsform betrifft die vorliegende Erfindung die Verwendung der erfindungsgemäßen DNA-Moleküle oder Teilen davon oder Derivaten, die durch Insertion, Deletion oder Substitution von diesen DNA-Molekülen abgeleitet sind, zur Transformation pro- und eukaryontischer Zellen. Um die Expression des 2-Oxoglutarat/Malat-Translokators in transformierten Zellen zu gewährleisten, können die erfindungsgemäßen DNA-Moleküle in Vektoren, vorzugsweise Plasmide, eingebracht werden und dabei mit Steuerelementen für die Expression in prokaryontischen oder eukaryontischen Zellen kombiniert werden (siehe Beispiele 3 und 5). Derartige Steuerelemente sind einerseits Transkriptions-Promotoren und andererseits Transkriptions-Terminatoren. Mit den Vektoren können eukaryontische Zellen transformiert werden mit dem Ziel der Expression einer translatierbaren Botenribonucleinsäure (mRNA), die die Synthese eines plastidären 2-Oxoglutarat/Malat-Translokators in den transformierten Zellen erlaubt, oder mit dem Ziel der Expression einer nicht-translatierbaren, invers orientierten ("anti-sense"), Botenribonucleinsäure, die die Synthese der endogenen 2-Oxoglutarat/Malat-Translokatoren verhindert. Zu diesem Zweck könnten auch kürzere Fragmente der erfindungsgemäßen DNA-Moleküle oder DNA-Moleküle, deren Sequenz einen relativ hohen Grad an Homologie (mehr als ca. 65 % Homologie) zu den Sequenzen der erfindungsgemäßen DNA-Moleküle aufweist, verwendet werden. Ebenso kann die Expression von endogenen Dicarbonsäuretranslokatoren durch die Expression eines für diesen Zweck konstruierten Ribozyms unter Verwendung der erfindungsgemäßen DNA-Moleküle inhibiert werden.

In einer weiteren Ausführungsform betrifft die vorliegende Erfindung daher rekombinante DNA-Moleküle, beispielsweise Plasmide, die eines der erfindungsgemäßen DNA-Moleküle umfassen, z.B. die Plasmide pBinAR-211 (DSM 9239), pEVP11-211 (DSM 9237) und pBSC-211 (DSM 9238) .

Gegenstand der Erfindung sind insbesondere rekombinante DNA-Moleküle, in denen ein erfindungsgemäßes DNA-Molekül mit DNA-Sequenzen verknüpft ist, die die Expression in pro- oder eukaryontischen Zellen erlauben.

In einer weiteren Ausführungsform betrifft die vorliegende Erfindung Bakterien, die erfindungsgemäße DNA-Moleküle oder erfindungsgemäße rekombinanten DNA-Moleküle enthalten.

Die erfindungsgemäßen DNA-Moleküle codieren für Proteine mit der Aktivität eines 2-Oxoglutarat/Malat-Translokators.

Es handelt sich dabei bevorzugt um Proteine aus dikotylen oder monokotylen Pflanzen, insbesondere aus Pflanzen der Familie der *Chenopodiaceae* und besonders bevorzugt aus *Spinacia oleracea*.

Die erfindungsgemäßen DNA-Moleküle können zur Herstellung derartiger Proteine verwendet werden. Dazu werden sie mit DNA-Sequenzen verknüpft, die die Transkription in pro- oder eukaryontischen Zellen gewährleisten. Die resultierenden rekombinanten Moleküle können anschließend in geeignete prooder eukaryontische Wirtszellen eingebracht und exprimiert werden. Das resultierende Protein kan nach den bekannten Methoden isoliert werden. Ferner ist es möglich, ein verkürztes Protein zu exprimieren, das keine Signalsequenzen enthält, die die Lokalisation des Proteins in der inneren Hüllmembran der Plastiden gewährleistet.

In einer weiteren bevorzugten Ausführungsform betrifft die vorliegende Erfindung die Verwendung der erfindungsgemäßen DNA-Moleküle zur Expression eines 2-Oxoglutarat/Malat-Translokators in pflanzlichen Zellen.

Durch die Expression einer RNA entsprechend den erfindungsgemäßen DNA-Molekülen, die für einen pflanzlichen 2-Oxoglutarat/Malat-Translokator codieren, ist eine Veränderung des pflanzlichen Stickstoffmetabolismus möglich, deren wirtschaftliche Bedeutung darin liegt, daß eine Verbesserung der Weiterleitung von 2-Oxoglutarat aus dem Cytosol in den Plastiden zu einer Veränderung des Verhältnisses von Kohlenhydraten (Zucker, Stärke, org. Säuren) und Fetten zugunsten der Stickstoffverbindungen (Aminosäuren, Proteine, evtl. Alkaloide) stattfindet. Es können somit Pflanzen erzeugt werden, die reicher an wertvollem Protein sind, jedoch einen geringeren Gehalt an Kohlenhydraten und Fetten aufweisen. Diese Modifikation steigert den ernährungsphysiologischen Wert von Pflanzen und damit auch deren wirtschaftlichen Wert.

Es sind bereits Verfahren zur genetischen Modifikation dikotyler und monokotyler Pflanzen bekannt (Gasser und Fraley, 1989, Science 244: 1293-1299; Potrykus, 1991, Ann. Rev. Plant Mol. Biol. Plant Physiol. 42: 205-225). Zur Expression von codierenden Sequenzen in Pflanzen müssen diese mit transkriptionell regulatorischen Elementen verknüpft werden. Solche Elemente, Promotoren genannt, sind bekannt (u.a. Köster-Töpfer et al., 1989, Mol. Gen. Genet. 219: 390-396). Ferner müssen die Codierregionen mit einem Transkriptionsterminations-Signal versehen werden, damit sie korrekt transkribiert werden können. Solche Elemente sind ebenfalls beschrieben (Gielen et al., 1989, EMBO J. 8, 23-29). Der transkriptionelle Startbereich kann sowohl nativ bzw. homolog als auch fremdartig bzw. heterolog zur Wirtspflanze sein. Terminationsbereiche sind gegeneinander beliebig austauschbar. Die DNA-Sequenz der Transkriptionsstart- und -terminationsregionen kann synthetisch hergestellt oder natürlich gewonnen sein oder eine Mischung aus synthetischen und natürlichen DNA-Bestandteilen enthalten.

Zur Vorbereitung der Einführung fremder Gene in höhere Pflanzen sind eine große Anzahl Clonierungsvektoren verfügbar, die ein Replikationssignal für *E. coli* und einen Marker beinhalten, der eine Selektion der transformierten Zellen erlaubt. Beispiele für Vektoren sind pBR322, pUC-Serien, M13mp-Serien, pACYC 184 usw. Je nach Einführungsmethode gewünschter Gene in die Pflanze können weitere DNA-Sequenzen erforderlich sein. Werden z.B. für die Transformation der Pflanze das Ti- oder Ri-Plasmid verwendet, so muß mindestens die rechte Begrenzung, häufig jedoch die rechte und die linke Begrenzung der Ti- und Ri-Plasmid T-DNA als Flankenbereich den einzuführenden Genen angefügt werden. Die Verwendung von T-DNA für die Transformation von Pflanzenzellen ist intensiv untersucht und ausreichend beschrieben (Hoekema, in: The Binary Plant Vector System, Offsetdrukkerij Kanters B-V. Ablasserdam, 1985, Chapter V; Fraley et al., Critic. Rev. Plant Sci. 4: 1-46; An et al., 1985, EMBO J. 4: 277-287). Ist die eingeführte DNA einmal im Genom integriert, so ist sie dort in der Regel stabil und bleibt auch in den Nachkommen der ursprünglich transformierten Zelle erhalten. Sie enthält normalerweise einen Selektionsmarker, der den transformierten Pflanzenzellen einen Resistenz gegenüber Bioziden oder Antibiotika wie Kanamycin, Bleomycin oder Hygromycin verleiht. Der individuell eingeführte Marker wird daher die Selektion transformierter Zellen gegenüber Zellen, denen die eingeführte DNA fehlt, gestatten.

Für die Einführung von DNA in eine pflanzliche Wirtszelle stehen neben der Transformation mit Hilfe von Agrobakterien viele andere Techniken zur Verfügung. Diese Techniken umfassen die Transformation von Protoplasten, die Mikroinjektion von DNA, die Elektroporation sowie ballistische Methoden. Aus dem transformierten Pflanzenmaterial können dann in einem geeigneten Selektionsmedium ganze Pflanzen regeneriert werden. Die so erhaltenen Pflanzen können dann mit gängigen molekularbiologischen Methoden auf die Anwesenheit der eingeführten DNA getestet werden. Diese Pflanzen können normal angezogen werden und mit Pflanzen, die die gleiche transformierte Erbanlage oder andere Erbanlagen besitzen, gekreuzt werden. Die daraus entstehenden hybriden Individuen haben die entsprechenden phänotypischen Eigenschaften.

Somit betrifft die vorliegende Erfindung auch transgene Pflanzenzellen, die erfindungsgemäße rekombinante DNA-Moleküle enthalten, sowie aus diesen transgenen Pflanzenzellen regenerierbare transgene Pflanzen und transgene Pflanzen die erfindungsgemäße Pflanzenzellen enthalten. Diese Pflanzenzellen und Pflanzen sind dadurch gekennzeichnet, daß sie ein rekombinantes DNA-Molekül im Genom integriert enthalten, das die Expression eines Proteins mit der Aktivität eines 2-Oxoglutarat/Malat-Translokators ermöglicht oder die Expression eines nicht-translatierbaren RNA-Moleküls, das die Synthese von 2-Oxoglutarat/Malat-Translokatoren inhibiert. Ferner betrifft die vorliegende Erfindung Samen der erfindungsgemäßen transgenen Pflanzen.

In einer weiteren Ausführungsform betrifft die vorliegende Erfindung die heterologe Expression der erfindungsgemäßen DNA-Moleküle in Pilzen z.B. in Spalthefen (Beispiele 3, 4 und Loddenkötter et al., 1993, Proc. Natl. Acad. Sci. USA 90: 2155-2159), insbesondere für Struktur-Funktionsstudien des 2-Oxoglutarat/Malat-Translokators, die letztendlich zur Entwicklung eines spezifischen Hemmstoffs für dieses Protein führen könnte. In diesem Zusammenhang ist beispielsweise auch an die Herbizidentwicklung zu denken, da die Hemmung eines Proteins mit Schlüsselfunktion im Stoffwechselgeschehen zwangsweise letal für die Pflanze wäre.

Daher können die erfindungsgemäßen DNA-Moleküle (oder Teile dieser Moleküle oder Derivate dieser Moleküle) auch in Vektoren eingebracht und dabei mit Steuerelementen für die Expression in Zellen von Pilzen, insbesondere Spalthefen, versehen werden (siehe Beispiel 3). Die Einführung des 2-Oxoglutarat/Malat-Translokators führt zu einer erheblichen Zunahme der durch Rekonstitution in künstliche Liposomen meßbaren Aktivität des 2-Oxoglutarat/Malat-Translokators in den rekombinanten Hefezellen. Hierbei ist anzumerken, daß Mitochondrien einen 2-Oxoglutarat/Malat-Translokator besitzen, der in seiner Substratspezifität ähnlich dem der Plastiden ist, jedoch in seiner DNA- und Aminosäuresequenz (Runswick et al., 1990, Biochemistry 29: 11033-11040) keine Ähnlichkeit zum plastidären Translokator aufweist. In rekombinaten Hefezellen ist eine bis zu 100-fach höhere Malat-Transportaktivität nachweisbar. Es ist somit denkbar, mit Hilfe der erfindungsgemäßen DNA-Moleküle (oder Teilen oder Derivaten dieser Moleküle) Hefezellen so zu verändern, daß sie einen veränderten Proteingehalt aufweisen. Hierbei käme der Vorteil eines plastidären Translokators aus Pflanzen zum tragen, nicht den endogenen Regulations- und Kompartiment-Adressierungsmechanismen der Spalthefen zu unterliegen. Mit hoher Wahrscheinlichkeit wäre die beschriebene Erhöhung der Malat-Transportaktivität in den Hefezellen durch eine Expression eines mitochondrialen 2-Oxoglutarat Translokators nicht zu erzielen. Diese Stämme wären insbesondere für die Futtermittelindustrie von herausragender Bedeutung.

Somit betrifft die Erfindung auch Pilzzellen, insbesondere Hefezellen, die ein erfindungsgemäßes DNA-Molekül oder rekombinantes DNA-Molekül enthalten.

Die erfindungsgemäßen DNA-Moleküle enthalten im codierenden Bereich Regionen, die in der Lage sind, das im Cytoplasma an Ribosomen synthetisierte Protein spezifisch zu Plastiden zu dirigieren und das Auftreten des Proteins in anderen Membransystemen der Zelle zu verhindern. Der Proteinbereich, der das durch die erfindungsgemäßen DNA-Moleküle codierte Protein zu Plastiden dirigiert, liegt innerhalb der ersten hundert Aminosäuren des Proteins, ist für die Transportfunktion des Proteins nicht erforderlich und wird nach erfolgreicher Insertion des Proteins in die Chloroplastenhüllmembran entfernt. Durch Austausch dieser "Plastidtargeting"-Sequenz gegen eine der bekannten "Targeting"-Sequenzen z.B. für Mitochondrien ließe sich das Translokator-Protein in ein anderes Membransystem eukaryontischer Zellen dirigieren und könnte dort möglicherweise die Transporteigenschaften über die respektive Membran verändern. Ebenso können die "Plastidtargeting"-Sequenz des 2-Oxoglutarat/Malat-Translokators, insbesondere die ersten 100 Aminosäuren, oder endogene Bereiche des reifen Proteins dazu genutzt werden, fremde Proteine (z.B. Proteine, die den aktiven oder passiven Transport von Metaboliten über Membranen katalysieren, Enzyme, bakterielle Transportproteine oder Transporter aus Hefen) in die Plastiden, insbesondere in die Plastidenhüllmembran, in das Plastidenstroma oder in die Thylakoide, pflanzlicher Zellen zu dirigieren.

Am 9. Juni 1994 wurden bei der als internationale Hinterlegungsstelle anerkannten Deutschen Sammlung von Mikroorganismen (DSM) in Braunschweig, Bundesrepublik Deutschland, entsprechend den Anforderungen des Budapester Vertrages folgende Plasmide in den angegebenen *E. coli* Stämmen hinterlegt:

| | | |
|---|---|---|
| Plasmid | pBinAR-211 | (DSM 9239) |
| Plasmid | pEVP11-211 | (DSM 9237) |
| Plasmid | pBSC-211 | (DSM 9238) |

### Beschreibung der Figuren:

- Figur 1:: Rekonstitution der Transportaktivität des in Hefe exprimierten rekombinanten 2-Oxoglutarat/Malat Translokators Gezeigt ist die Aufnahme von [¹⁴C]Malat in rekonstituierte und *S. pombe*-Zellmembranen enthaltende Liposomen.
- Figur 2:: Schematische Darstellung der Clonierung des Plasmids pEVP11-211 (Figur 2A) und des Plasmids pBinAR-211 (Figur 2B)
- Figur 3:: Adressierung des 2-Oxoglutarat/Malat-Translokator Vorstufenproteins zu den Chloroplasten und energieabhängige Insertion des reifen Proteins in die innere Hüllmembran

Die SDS-Polyacrylamidgelelektrophorese wurde wie in Beispiel 6 beschrieben durchgeführt:
- Spur 1:: *in vitro* translatiertes Vorstufenprotein
- Spuren 2-4:: Dunkelbedingungen
- Spuren 5-9:: Lichtbedingungen
- Spur 2:: ohne ATP
- Spur 3:: plus 2 mM ATP
- Spur 4:: plus 2 mM ATP, anschließende ProteaseBehandlung (100 µg/ml)
- Spur 5:: ohne ATP
- Spur 6:: plus 2 mM ATP
- Spur 7:: plus 2 mM ATP, anschließende Protease-Behandlung (100 µg/ml)
- Spur 8:: plus Entkoppler (10 µM CCCP)
- Spur 9:: plus 2 mM ATP; Protease-vorbehandelte Chloroplasten (30 µg/ml)

Zum besseren Verständnis der dieser Erfindung zugrundeliegenden Beispiele werden einige der angewendeten Methoden im folgenden näher erläutert.

### 1. Clonierungsverfahren

Zur Clonierung wurden der Phage Lambda gt10 sowie der Vektor pBluescript II KS (pBSC) (Short et al., 1988, Nucl. Acids Res. 16: 7583-7600) verwendet.

Für die Transformation von Hefen wurde der Vektor pEVP11 (Russel und Nurse, 1986, Cell 45: 145-153) verwendet.

Für die Pflanzentransformation wurden die Genkonstruktionen in den binären Vektor pBinAR (Höfgen und Willmitzer, 1990, Plant Sci. 66: 221-230) cloniert.

### 2. Bakterien- und Hefestämme

Für den pBluescriptKS (pBSC) Vektor sowie für pEVP11- und pBinAR-Konstrukte wurden die *E. coli* Stämme DH5α (Hanahan et al., 1983, J. Mol. Biol. 166: 557-580) und TG1 (Gibson, 1984, Ph.D. Thesis, Cambridge University, England) verwendet.

Die Transformation der pBinAR-Konstrukte in Tabakpflanzen wurde mit Hilfe des *Agrobacterium tumefaciens*-Stammes LBA4404 (Bevan, 1984, Nucl. Acids Res. 12: 8711-8720) durchgeführt.

### 3. Transformation von Agrobacterium tumefaciens

Der Transfer der DNA in die Agrobakterien erfolgte durch direkte Transformation nach der Methode von Höfgen und Willmitzer (1988, Nucl. Acids Res. 16: 9877). Die Plasmid-DNA transformierter Agrobakterien wurde nach der Methode von Birnboim und Doly (1979, Nucl. Acids Res. 7: 1513-1523) isoliert und nach geeigneter Restriktionsspaltung gelektrophoretisch auf Richtigkeit und Orientierung analysiert.

### 4. Pflanzentransformation

Pro Transformation wurden 15 kleine mit Schmirgelpapier und Skalpell verwundete Blätter einer Tabak-Sterilkultur in 10 ml MS-Medium mit 2 % Saccharose gelegt, welches 100 µl einer unter strenger Selektion gewachsenen, transformierten *Agrobacterium tumefaciens*-Übernachtkultur enthielt. Nach 15 minütigem leichtem Schütteln wurden die Blätter auf MS-Medium mit 1,6 % Glucose, 2 mg/l Zeatinribose, 0,02 mg/l Naphthylessigsäure, 0,02 mg Giberellinsäure, 500 mg/l Betabactyl®, 15 mg/l Hygromycin und 0,8 % Bacto-Agar ausgelegt. Nach einwöchiger Inkubation bei 25°C und 3.000 Lux Beleuchtungsstärke wurde die Betabactylkonzentration im Medium um die Hälfte reduziert.

Die Beispiele erläutern die Erfindung.

### Beispiel 1

### Isolation von Peptidfragmenten des 2-Oxoglutarat/Malat-Translokators und Erstellung von Sonden für die Hybridisationsdurchmusterung von cDNA Bibliotheken

Gereinigtes 2-Oxoglutarat/Malat-Translokatorprotein (Menzlaff u. Flügge, 1993, Biochim. Biophys. Acta 1147: 13-18) wurde in präparativen SDS-Polyacrylamidgelen (Laemmli, 1970, Nature 227: 680-685) von verbleibenden Verunreinigungen getrennt und nach Detektion des Proteins durch Anfärbung mit Kupfersulfat (Lee at al., 1987, Anal. Biochem. 166: 308-312) aus dem Gel ausgeschnitten und in der Gelmatrix mit Endoproteinase LysC verdaut (Eckerskorn und Lottspeich, 1989, Chromatographia 28: 92-94). Die resultierenden Peptide wurden aus dem Gel eluiert und mittels HPLC getrennt. Die Aminosäuresequenz der gereinigten Peptidfraktionen wurde durch automatisierten Edman-Abbau in der Gasphase bestimmt (Eckerskorn et al., 1988, Eur. J. Biochem. 176: 509-519). Aus der Aminosäuresequenz von drei Peptiden wurden degenerierte Oligonucleotidsequenzen, codierend diese Aminosäuresequenzen, abgeleitet und die respektiven Oligonucleotide durch *in vitro* DNA-Synthese hergestellt. Für den Einsatz als Sonde wurden die Oligonucleotide durch Anhängen einer ³²P-Phosphatgruppe an das 5'-Ende mittels einer Oligonucleotidkinase radioaktiv markiert.

### Beispiel 2

### Clonierung des 2-Oxoglutarat/Malat-Translokators aus Spinat

Aus jungen Blättern von in Hydrokultur gewachsenen Spinatpflanzen wurde poly-A⁺-RNA isoliert und ausgehend hiervon eine cDNA-Bibliothek im Vektor Lambda gt10 angelegt (Flügge et al., 1989, EMBO J. 8: 39-46). Ca. 300.000 Clone dieser Bibliothek wurden mit nach Endoproteinase LysC-Peptidfragmenten des gereinigten 2-Oxoglutarat/Malat-Translokators modellierten synthetischen Oligonucleotiden sondiert (siehe Beispiel 1). Positiv reagierende Clone wurden nach Standardverfahren gereinigt und nach Präparation der amplifizierten Phagen-DNA aus den gereinigten Plaques wurde durch *EcoRI*-Restriktionsverdau die Insertion codierend für den 2-Oxoglutarat/Malat-Translokator erhalten und durch Southern-Blot Analyse, mit den oben erwähnten Oligonucleotiden als Sonde, verifiziert. Nach Umclonierung der Insertionen der Phagen-DNA in den Vektor pBluescript (pBSC) wurden die Clone durch Bestimmung der DNA-Sequenz analysiert (Dideoxymethode: Sanger et al., 1977, Proc. Natl. Acad. Sci. USA 74: 5463-5467) und aus dieser DNA-Sequenz die Primärstruktur des 2-Oxoglutarat/Malat-Translokators abgeleitet. Die Sequenz der zum Sondieren der cDNA-Bibliothek benutzten Oligonucleotide bzw. Peptide konnte wiedergefunden werden.

### Beispiel 3

### Expression des 2-Oxoglutarat/Malat Translokators-aus Spinat in der Spalthefe Schizosaccharomyces pombe

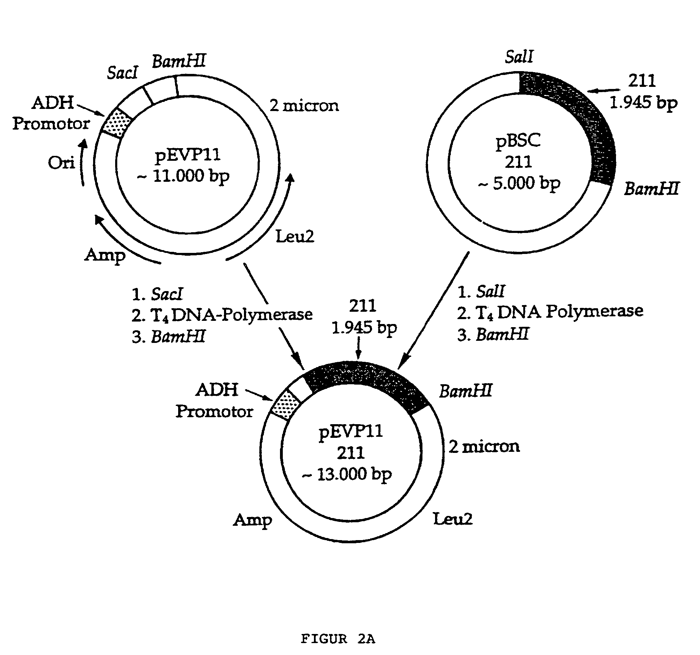

Das oben erwähnte Plasmid pBluescript (pBSC), enthaltend die Insertion codierend für den 2-Oxoglutarat/Malat-Translokator, wurde mit der Endonuclease *Sal*I linearisiert und die resultierenden Überhänge mit Hilfe des Enzyms T4-DNA Polymerase geglättet. Daraufhin wurde die Insertion durch einen weiteren Restriktionsverdau mit *Bam*HI aus dem Vektor herausgeschnitten und durch Elektrophorese isoliert. Das so erhaltene Fragment wurde in den Hefe-Expressionsvektor pEVP11, der zunächst mit *Sac*I linearisiert, die Enden mit T4-DNA Polymerase geglättet und dann erneut mit *Bam*HI geschnitten wurden, gerichtet eingesetzt (siehe auch Figur 2A) und nach Amplifikation des Konstrukts in *E. coli* in durch LiCl/PEG kompetent gemachte (Ito et al., 1983, J. Bact. 153: 163-168) Leucinsynthesedefiziente *S. pombe* Zellen transformiert. Transformanten wurden durch Selektion auf Minimalmedium ohne Leucin selektiert, da das pEVP11-211 Konstrukt den Hefezellen die Fähigkeit zum Wachstum auf leucinfreiem Medium verleiht.

### Beispiel 4

### Messung der Malat-Transportaktivität in rekombinaten Hefelinien

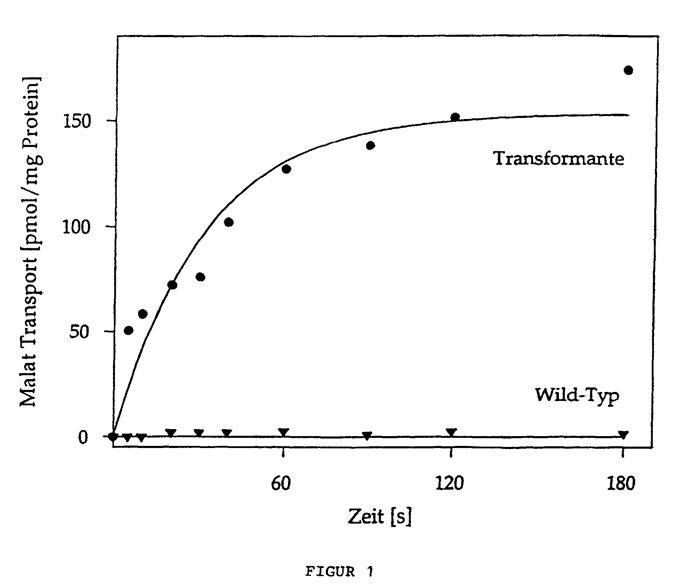

Mit dem pEVP11-211 Plasmid transformierte Hefezellen (siehe Figur 2A, SP-DC3-Zellen) wurden in Minimalmedium bis zu einer optischen Dichte bei 600 nm von 1.0 angezogen und durch Zentrifugation bei 3.000 x g für 5 min geerntet. Anschließend wurden die Zellen durch kräftiges Schütteln mit 1/2 vol (bezogen auf die Zellen) Glasperlen aufgebrochen und Glasperlen und Zellbruchstücke durch Zentrifugation (600 g für 1 min) abgetrennt. Der Überstand wurde auf eine Konzentration von 0.5 % (Gewicht/Volumen) Triton X-100 eingestellt, mit dem gleichen Volumen Liposomen versetzt und die resultierenden Proteoliposomen sofort in flüssigem Stickstoff eingefroren. Die Liposomen wurden zuvor durch Beschallen von Sojabohnen-Phospholipid (20 mg/ml) für 10 min bei 4°C in Gegenwart von 200 mM Tricine-NaOH (pH 7,6), 40 mM Malat und 60 mM Kaliumgluconat hergestellt. Nach dem Auftauen der Proteoliposomen und erneutem Beschallen der Suspension mit 10 Pulsen à 1 s wurden die Proteoliposomen vom umgebendem Medium durch Größenausschlußchromatographie auf Sephadex G-25, das zuvor mit 10 mM Tricine-NaOH (pH 7,6), 100 mM Natriumgluconat und 50 mM Kaliumgluconat equilibriert worden war, abgetrennt. Die eluierten Proteoliposomen wurden für die Messung der Malat-Transportaktivität eingesetzt. Die Messung wurde nach der von Menzlaff und Flügge (1993, Biochim. Biophys. Acta 1147: 13-18) beschriebenen "Inhibitor-Stop" Methode durchgeführt.
Die Malat-Transportaktivität in den pEVP11-211 Transformanten wurde mit der Malat-Transportaktivität von Transformanten verglichen, die lediglich mit dem Vektor pEVP11 ohne die 211-Insertion transformiert waren. Es zeigte sich, das die Malat-Transportaktivität in den pEVP11-211 Transformanten (gemessen in pmol transportiertes ¹⁴C-Malat/mg Protein * Stunde) 100-fach höher war als in pEVP11 Kontroll-Transformanten. (Figur 1). Zudem konnte gezeigt werden, daß das rekombinante Translokatorprotein im Vergleich zu dem authentischen Protein der Chloroplastenmembran identische Transport-Charakteristika aufweist. Dies ist in der folgenden Tabelle dargestellt.

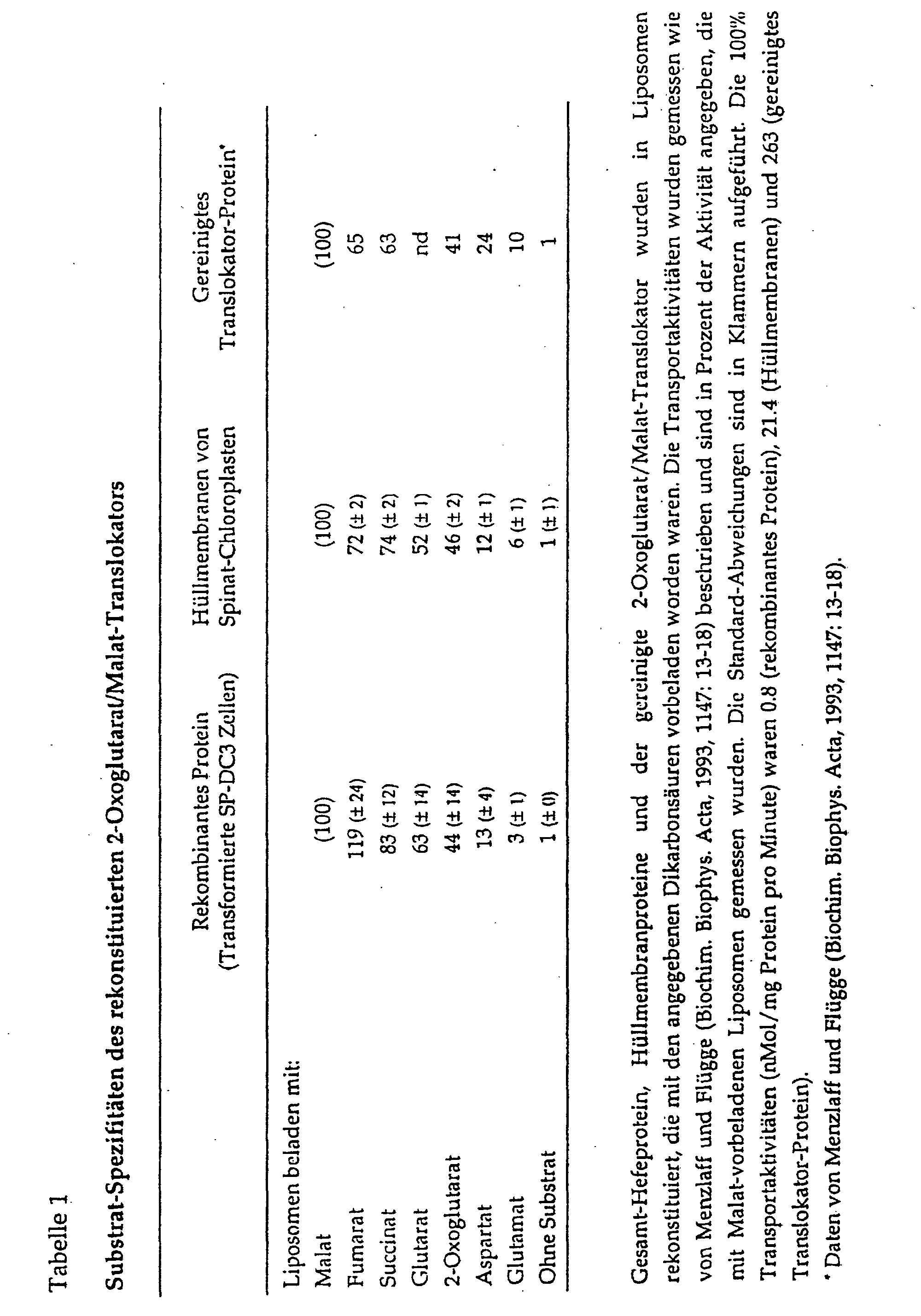

### Beispiel 5

### Transformation von Pflanzen mit einer Konstruktion zur Überexpression der Codierregion des 2-Oxoglutarat/Malat-Translokators

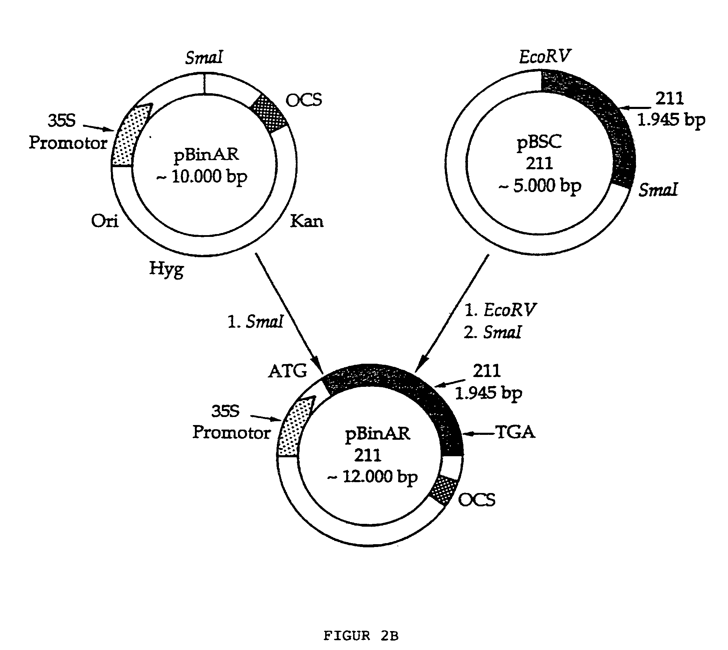

Aus dem Vektor pBluescript-211 (pBSC-211), der als Insertion die cDNA für den 2-Oxoglutarat/Malat-Translokator aus Spinat enthielt (siehe Beispiel 2, Figur 2B) wurde die Insertion durch Restriktionsverdau mit *Eco*RV und *Sma*I isoliert und in den Vektor pBinAR (Höfgen u. Willmitzer, 1990, Plant Sci. 66: 221-230) der zuvor mit dem Enzym *Sma*I geschnitten worden war, cloniert. Nach Amplifikation des resultierenden Konstrukts pBinAR-211 in *E. coli* wurde das Konstrukt in Agrobakterien transformiert und diese dann zur Infektion von Blattsegmenten von Tabak und Kartoffel eingesetzt.
Die erhaltenen Transformanten wurden mit Hilfe von Southern-Blot Analysen auf die Präsenz des intakten, nicht rearrangierten chimären Gens untersucht. Mit Hilfe der "Gesamtblatt-Rekonstiutionsmethode" (Flügge und Weber, 1994, Planta, 194: 181-185) wurde die Malattransportaktivität im Vergleich zu Kontrolltransformanten (transformiert mit Vektor pBinAR ohne Insertion) untersucht, ebenso das C/N-Verhältnis, Photosyntheserate, Transpiration und Wachstum.

### Beispiel 6

### Adressierung des 2-Oxoglutarat/Malat-Translokator Vorstufenproteins zu den Chloroplasten und energieabhängige Insertion

### des reifen Proteins in die innere Hüllmembran.

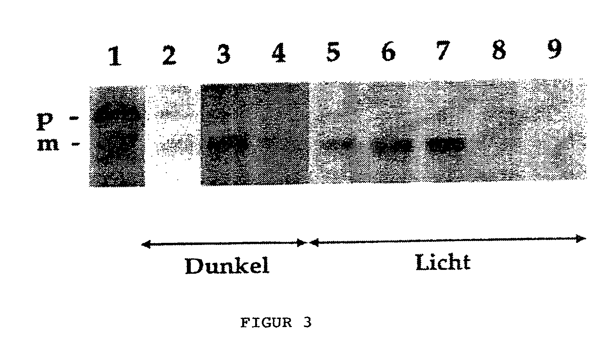

Die *in vitro* Transkription des mit *Sma*I linearisierten Plasmids pBSC-211 wurde unter Verwendung von T3 RNA-Polymerase nach den Angaben des Herstellers (Pharmacia) durchgeführt.
Die anschließende *in vitro* Translation erfolgte im Retikulozyten-Lysat (Boehringer-Mannheim) und der postribosomale Überstand wurde für Proteintransporte in intakte Spinat-Chloroplasten benutzt. Das Experiment wurde im Dunkeln bzw. im Licht durchgeführt; der Ansatz enthielt Import-Puffer (Flügge et al., 1989, EMBO J. 8: 39-46), intakte Spinat-Chloroplasten (entsprechend 200 mg Chlorophyll) und verschiedene, in der Legende zu Figur 3 aufgeführte Zugaben (Gesamtvolumen: 300 ml). Nach 15 min bei 25 °C wurde die Chloroplasten gewaschen und die Hüllmembranen isoliert (Flügge et al., 1989, EMBO J. 8: 39-46). Sie wurden über eine SDS-Polyacrylamid Gelelektrophorese (Laemmli, 1970, Nature 227: 680-685) und anschließende Fluorographie (Bonner und Laskey, 1974, Eur. J. Biochem. 46: 84-88) analysiert (Figur 3 ) . Spur 1 zeigt das *in vitro* translatierte Vorstufenprotein (p). Spuren 2-4: Dunkelbedingungen, Spuren 5-9: Lichtbedingungen. Unter allen Bedingungen dirigiert die Präsequenz des 2-Oxoglutarat/Malat-Translokators das anhängende reife Protein korrekt zu seiner Zielmembran, der inneren Chloroplasten-Hüllmembran; sie wird während des Import- Prozesses durch eine spezifische Protease abgespalten, das reife Protein (m) ensteht. Im Dunkeln wird die Insertion des Translokators durch die Zugabe von ATP energetisiert (Spuren 3 und 4). In Abwesenheit von ATP (Spur 1) wird kein Import beobachtet. Im Licht kann die Energie für den Import des Proteins in Form von ATP über die photosynthetische Phosphorylierung bereitgestellt werden; der Import ist unter diesen Bedingungen unabhängig von äußerem ATP (Spur 5), kann aber durch von außen zugegebenes ATP gesteigert werden (Spur 6). Wird die photosynthetische Phosphorylierung und die damit verbundene Produktion von ATP durch die Zugabe eines Entkopplers wie CCCP verhindert, ist auch der Proteinimport blockiert (Spur 8). Das reife Protein liegt eingebaut in die innere Membran vor: Zugabe von Proteasen (z.B. Thermolysin), die die äußere Hüllmembran nicht permeieren können, vermögen das in die innere Membran eingebaute reife Protein nicht anzugreifen (Spuren 4 und 7). Eine Vorbehandlung der Chloroplasten mit einer Protease (z.B. Thermolysin) führt zu einem völligen Verlust der Bindung und des Importes des Translokators (Spur 9). Dies zeigt, daß die Präsequenz ("Targeting"-Sequenz) des Translokators in einem ersten Schritt zunächst spezifisch an Rezeptoren der äußeren Membran gebunden werden muß. Erst dann können dann die weiteren Schritte der Protein-Insertion erfolgen.

## Patentansprüche

1. DNA-Molekül, das für einen plastidären 2-Oxoglutarat/Malat-Translokator codiert, ausgewählt aus der Gruppe bestehend aus
(a) DNA-Molekülen, die für ein Protein mit der unter SEQ ID NO:1 dargestellten Aminosäuresequenz codieren;
(b) DNA-Molekülen, die den unter SEQ ID NO:1 dargestellten codierenden Bereich aufweisen;
(c) DNA-Molekülen, deren komplementärer Strang mit einem der unter (a) oder (b) genannten DNA-Molekülen hybridisiert; und
(d) DNA-Molekülen, deren Sequenz aufgrund des genetischen Codes degeneriert ist im Vergleich zu den Sequenzen der unter (a), (b) oder (c) genannten DNA-Moleküle.

2. DNA-Molekül, das zu einem DNA-Molekül nach Anspruch 1 komplementär ist.

3. Rekombinantes DNA-Molekül enthaltend ein DNA-Molekül nach Anspruch 1.

4. Rekombinantes DNA-Molekül nach Anspruch 3, in dem das DNA-Molekül nach Anspruch 1 mit DNA-Sequenzen verknüpft ist, die die Transkription in pro- oder eukaryotischen Zellen gewährleisten.

5. Rekombinantes DNA-Molekül nach Anspruch 4, das die Expression einer translatierbaren mRNA, die für ein Protein mit der Aktivität eines 2-Oxoglutarat/Malat-Translokators codiert, gewährleistet.

6. Rekombinantes DNA-Molekül nach Anspruch 4, das die Expression einer nicht-translatierbaren RNA gewährleistet, die mittels eines anti-sense-Effektes oder einer Ribozymaktivität die Synthese eines oder mehrerer 2-Oxoglutarat/Malat-Translokatoren inhibitiert.

7. Plasmid pBSC-211, hinterlegt als DSM 9238 im E.coli-Stamm TG1 pBSC-211.

8. Plasmid pEVP11-211, hinterlegt als DSM 9237 im E.coli-Stamm TG1 pERP11-211.

9. Plasmid pBinAR-211, hinterlegt als DSM 9239 im E.coli-Stamm TG1 pBINAR-211.

10. Bakterien, enthaltend ein DNA-Molekül nach Anspruch 1, ein rekombinantes DNA-Molekül nach einem der Ansprüche 3 bis 6 oder ein Plasmid nach einem der Ansprüche 7 bis 9.

11. Pilze enthaltend ein DNA-Molekül nach Anspruch 1, ein rekombinantes DNA-Molekül nach einem der Ansprüche 3 bis 6 oder ein Plasmid nach Anspruch 8.

12. Transgene Pflanzenzelle enthaltend ein rekombinantes DNA-Molekül nach Anspruch 6.

13. Transgene Pflanzenzelle enthaltend das Plasmid nach Anspruch 9.

14. Transgene Pflanzenzelle enthaltend ein DNA-Molekül nach Anspruch 1, wobei dieses verknüpft ist mit einem transkriptionellen Startbereich, der heterolog zur Wirtspflanze ist.

15. Transgene Pflanzen enthaltend Pflanzenzellen nach Anspruch 12.

16. Transgene Pflanze enthaltend Pflanzenzellen nach Anspruch 13.

17. Transgene Pflanze enthaltend Pflanzenzellen nach Anspruch 14.

18. Verwendung von DNA-Molekülen nach Anspruch 1 zur Isolierung von DNA-Molekülen, die für ein Polypeptid codieren, das die biologische Aktivität eines Dicarbonsäuretranslokators, insbesondere eines 2-Oxoglutarat/Malat-Translokators oder eines Glutamat/Malat-Translokators besitzt.

19. Verwendung von Teilen eines DNA-Moleküls nach Anspruch 1, die eine Sequenz umfassen, die für die ersten 100 Aminosäuren eines 2-Oxoglutarat/Malat-Translokators codiert, der von einem DNA-Molekül nach Anspruch 1 codiert wird, wobei diese Teile als "targeting"-Sequenzen dienen, um mit Hilfe dieser Sequenzen prokaryotische oder eukaryotische Proteine in die Plastidenhüllmembran, in das Plastidenstroma oder in die Thylakoide zu dirigieren.

20. Verwendung von Teilen von DNA-Molekülen nach Anspruch 1, wobei diese Teile eine codierende Region enthalten, die für ein reifes Protein mit der biologischen Aktivität eines 2-Oxoglutarat/Malat-Translokators codiert, und wobei das reife Protein nicht die in den ersten 100 Aminosäuren lokalisierte "targeting"-Sequenz des 2-Oxoglutarat/Malat-Translokators, der von einem DNA-Molekül nach Anspruch 1 codiert wird, enthält, zur Kombination mit "targeting"-Sequenzen für andere Zellkompartimente oder zelluläre Membransysteme, wodurch das reife Protein in andere Kompartimente oder Membransysteme dirigiert wird.

21. Verwendung von DNA-Molekülen nach Anspruch 1 zur Identifizierung von Substanzen, die eine inhibierende Wirkung auf den Transport von Dicarbonsäuren oder Glutamat über die innere Plastidenhüllmembran haben.

## Claims

1. A DNA molecule coding for a plastidic 2-oxoglutarate/malate translocator, selected from the group consisting of
(a) DNA molecules coding for a protein with the amino acid sequence shown under SEQ ID NO:1;
(b) DNA molecules having the coding region shown under SEQ ID NO:1;
(c) DNA molecules, the complementary strand of which hybridises to one of the DNA molecules indicated under (a) or (b); and
(d) DNA molecules, the sequence of which is degenerated due to the genetic code in comparison with the sequences of the DNA molecules indicated under (a), (b) or (c).

2. A DNA molecule which is complementary to a DNA molecule according to claim 1.

3. A recombinant DNA molecule containing a DNA molecule according to claim 1.

4. The recombinant DNA molecule according to claim 3, wherein the DNA molecule according to claim 1 is linked to DNA sequences which ensures the transcription in prokaryotic or eukaryotic cells.

5. The recombinant DNA molecule according to claim 4 which ensures the expression of a translatable mRNA which codes for a protein with the activity of a 2-oxoglutarate/malate trans locator.

6. The recombinant DNA molecule according to claim 4 which ensures the expression of a non-translatable RNA inhibiting the synthesis of one or more 2-oxoglutarate/malate translocators by means of an anti-sense effect or a ribozyme activity.

7. The plasmid pBSC-211 deposited as DSM 9238 in the E. coli strain TG1 pBSC-211.

8. The plasmid pEVP11-211 deposited as DSM 9237 in the E. coli strain TG1 pERP11-211.

9. The plasmid pBinAR-211 deposited as DSM 9239 in the E. coli strain TG1 pBINAR-211.

10. Bacteria containing a DNA molecule according to claim 1, a recombinant DNA molecule according to any one of claims 3 to 6 or a plasmid according to any one of claims 7 to 9.

11. Fungi containing a DNA molecule according to claim 1, a recombinant DNA molecule according to any one of claims 3 to 6 or a plasmid according to claim 8.

12. Transgenic plant cells containing a recombinant DNA molecule according to claim 6.

13. A transgenic plant cell containing the plasmid according to claim 9.

14. Transgenic plant cell containing a DNA molecule according to claim 1, wherein said DNA molecule is linked with a transcriptional starting region which is heterologous to the host plant.

15. Transgenic plants containing plant cells according to claim 12.

16. A transgenic plant containing plant cells according to claim 13.

17. A transgenic plant containing plant cells according to claim 14.

18. Use of DNA molecules according to claim 1 in order to isolate DNA molecules coding for a polypeptide with the biologic activity of a dicarboxylic acid translocator, particularly of a 2-oxoglutarate/malate trans locator or of a glutamate/malate translocator.

19. Use of parts of a DNA molecule according to claim 1 comprising a sequence which codes for the first 100 amino acids of a 2-oxoglutarate/malate translocator which is encoded by a DNA molecule according to claim 1, wherein said parts serve as targeting sequences, in order to direct prokaryotic or eukaryotic proteins into the plastid coating membrane, into the plastid stroma or into the thylakoids by means of said sequences.

20. Use of the parts of the DNA molecules according to claim 1, wherein these parts contain a coding region which codes for a mature protein with the biologic activity of a 2-oxoglutarate/malate translocator and wherein the mature protein does not contain the targeting sequence of the 2-oxoglutarate/malate translocator localised in the first 100 amino acids, the 2-oxoglutarate/malate translocator being encoded by a DNA molecule according to claim 1, for combination with targeting sequences for other cell compartments or cellular membrane systems whereby the mature protein is directed into other compartments or membrane systems.

21. Use of the DNA molecules according to claim 1 for identifying substances having an inhibitory effect on the transport of dicarboxylic acids or glutamate by their inner plastid coating membrane.

## Revendications

1. Molécule d'ADN qui code pour un translocateur de plastide 2-oxoglutarate /malate, choisie dans le groupe constitué par :
a) des molécules d'ADN, qui codent pour une protéine ayant la séquence d'acides aminés représentée par SEQ ID NO : 1 ;
b) des molécules d'ADN, qui présentent le domaine codant représenté par SEQ ID NO : 1 ;
c) des molécules d'ADN, dont le brin complémentaire hybride avec une des molécules d'ADN désignées sous (a) ou (b) ;
d) des molécules d'ADN, dont la séquence est dégénérée sur la base du code génétique par rapport aux séquences des molécules d'ADN désignées sous (a), (b) ou (c).

2. Molécule d'ADN, qui est complémentaire d'une molécule d'ADN selon la revendication 1.

3. Molécule d'ADN recombinante contenant une molécule d'ADN selon la revendication 1.

4. Molécule d'ADN recombinante selon la revendication 3, dans laquelle la molécule d'ADN selon la revendication 1 est fusionnée avec des séquences d'ADN qui effectuent la transcription dans des cellules pro- ou eucaryotes.

5. Molécule d'ADN recombinante selon la revendication 4, qui effectue l'expression d'un ARNm traduisible qui code pour une protéine ayant l'activité d'un translocateur 2-oxoglutarate / malate.

6. Molécule d'ADN recombinante selon la revendication 4, qui effectue l'expression d'un ARN non traduisible, qui inhibe la synthèse d'un ou plusieurs translocateurs 2-oxoglutarate / malate au moyen d'un effet anti-sens ou d'une activité ribozymale.

7. Plasmide pBSC-211, déposé en tant que DSM 9238 dans la souche E. Coli TG1 pBSC-211.

8. Plasmide pEVP 11-211, déposé en tant que DSM 9237 dans la souche E. Coli TG1 pERP 11-211.

9. Plasmide pBinAR-211, déposé en tant que DSM 9239 dans la souche E. Coli TG1 pBINAR-211.

10. Bactérie, comprenant une molécule d'ADN selon la revendication 1, une molécule d'ADN recombinante selon l'une des revendications 3 à 6 ou un plasmide selon l'une quelconque des revendications 7 à 9.

11. Champignon comprenant une molécule d'ADN selon la revendication 1, une molécule d'ADN recombinante selon l'une des revendications 3 à 6 ou un plasmide selon la revendication 8.

12. Cellules végétales transgéniques comprenant une molécule d'ADN recombinante selon la revendication 6.

13. Cellules végétales transgéniques comprenant le plasmide selon la revendication 9.

14. Cellules végétales transgéniques comprenant une molécule d'ADN selon la revendication 1, celle-ci étant fusionnée avec un domaine d'initiation de la transcription hétérologue par rapport à la plante hôte.

15. Plante transgénique comprenant des cellules végétales selon la revendication 12.

16. Plante transgénique comprenant des cellules végétales selon la revendication 13.

17. Plante transgénique comprenant des cellules végétales selon la revendication 14.

18. Utilisation de molécules d'ADN selon la revendication 1 pour l'isolement de molécules d'ADN, qui codent pour un polypeptide qui possède l'activité biologique d'un transcolateur d'acide dicarboxylique, en particulier d'un transcolateur 2-oxoglutarate / malate ou d'un transcolateur glutamate / malate.

19. Utilisation de parties d'une molécule d'ADN selon la revendication 1, qui englobent une séquence qui code pour les 100 premiers acides aminés d'un transcolateur 2-oxoglutarate / malate, lequel est codé par une molécule d'ADN selon la revendication 1, ces parties servant en tant que séquences « de ciblage », pour diriger avec l'aide de ces séquences des protéines procaryotes ou eucaryotes dans la membrane du plastide, dans le stroma du plastide ou dans la thylacoïde.

20. Utilisation de parties de molécules d'ADN selon la revendication 1, ces parties comprenant une région codante qui code pour une protéine mature ayant l'activité biologique d'un transcolateur 2-oxoglutarate / malate, la protéine mature ne contenant pas les séquences de « ciblage » localisées dans les 100 premiers acides aminés du transcolateur 2-oxoglutarate / malate, lequel est codé par une molécule d'ADN selon la revendication 1, pour la combinaison avec des séquences de « ciblage » pour d'autres compartiments cellulaires ou systèmes membranaires cellulaires, dans laquelle la protéine mature est dirigée dans d'autres compartiments ou systèmes membranaires.

21. Utilisation de molécules d'ADN selon la revendication 1 pour l'identification de substances qui ont une action d'inhibition du transport d'acides dicarboxyliques ou du glutamate au travers de la membrane interne du plastide.


### SEQUENZPROTOKOLL

(1) ALLGEMEINE ANGABEN:
   (i) ANMELDER:
      (A) NAME: Institut fuer Genbiologische Forschung Berlin GmbH
      (B) STRASSE: Ihnestr. 63
      (C) ORT: Berlin
      (E) LAND: DE
      (F) POSTLEITZAHL: 14195
      (G) TELEFON: (030) 83 00 07-0
      (H) TELEFAX: (030) 83 00 07-36
   (ii) BEZEICHNUNG DER ERFINDUNG: DNA-Molekuele, die fuer einen plastidaeren 2-Oxogluarat/Malat-Translokator codieren
   (iii) ANZAHL DER SEQUENZEN: 2
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
   (vi) DATEN DER URANMELDUNG:
      (A) ANMELDENUMMER: DE P 44 20 782.4
      (B) ANMELDETAG: 15-JUN-1994
(2) ANGABEN ZU SEQ ID NO: 1:
   (i) SEQUENZKENNZEICHEN:
      (A) LÄNGE: 1945 Basenpaare
      (B) ART: Nucleotid
      (C) STRANGFORM: Einzelstrang
      (D) TOPOLOGIE: linear
   (ii) ART DES MOLEKÜLS: cDNA
   (vi) URSPRÜNLICHE HERKUNFT:
      (A) ORGANISMUS: Spinacia oleracea
   (vii) UNMITTELBARE HERKUNFT:
      (A) BIBLIOTHEK: cDNA-Bibliothek in Phage Lambda gt10
   (ix) MERKMAL:
      (A) NAME/SCHLÜSSEL: CDS
      (B) LAGE:14..1720
      (D) SONSTIGE ANGABEN:/note=
         "2-Oxoglutarat/Malat-Translokator"
   (xi) SEQUENZBESCHREIBUNG: SEQ ID NO: 1:
   GGAGCCTTCA ACA ATG GCG TCC ATG GCT CTG AGT CTA ACT TCA TCT CCT      49
                  Met Ala Ser Met Ala Leu Ser Leu Thr Ser Ser Pro
                   1               5                  10
   ACC TAC TCT CTC TCC TTC AGA TCT CTC CCT TCT CTC AAA CCT CTC TCC     97
   Thr Tyr Ser Leu Ser Phe Arg Ser Leu Pro Ser Leu Lys Pro Leu Ser
           15                  20                  25
   AAA TCT CAA CCC TCC ATT TCC CTC CCT TCT CTC AGA TCC AAC GCT TCA    145
   Lys Ser Gln Pro Ser Ile Ser Leu Pro Ser Leu Arg Ser Asn Ala Ser
       30                  35                  40
   AAA TCT CCA TCT CTC TCA CAC AAA CAC TTC CTC TCT CCT CCT TCT CTT    193
   Lys Ser Pro Ser Leu Ser His Lys His Phe Leu Ser Pro Pro Ser Leu
   45                  50                  55                  60
   CTC CTC CCT CAC AAA CTC AAA CCC ATT TCA GCT TCT TCC CCA ACA AAC    241
   Leu Leu Pro His Lys Leu Lys Pro Ile Ser Ala Ser Ser Pro Thr Asn
                   65                  70                  75
   CCA CCT CCA CCT CCA GCT CCA GTC CCC TCC CCA GCT CCA GTT TCA GCT    289
   Pro Pro Pro Pro Pro Ala Pro Val Pro Ser Pro Ala Pro Val Ser Ala
               80                  85                  90
   CCA GCT CAA GTT CAG CCA TGG CAG GGA GCT TCA ATC AAA CCT CTA CTC    337
   Pro Ala Gln Val Gln Pro Trp Gln Gly Ala Ser Ile Lys Pro Leu Leu
           95                  100                 105
   GCA TCA ATC TTA ACA GGT GTC ATA ATC TGG TTC ATC CCT ACA CCA GAG    385
   Ala Ser Ile Leu Thr Gly Val Ile Ile Trp Phe Ile Pro Thr Pro Glu
       110                 115                 120
   GGA GTC TCC AGA AAC GCA TGG CAA CTC CTC GCA ATC TTC CTC TCC ACC    433
   Gly Val Ser Arg Asn Ala Trp Gln Leu Leu Ala Ile Phe Leu Ser Thr
   125                 130                 135                 140
   ATT GTT GGA ATC ATC ACT CAA CCT TTA CCT TTG GGT GCC GTT GCA TTG    481
   Ile Val Gly Ile Ile Thr Gln Pro Leu Pro Leu Gly Ala Val Ala Leu
                   145                 150                 155
   ATG GGG TTA GGC GCT TCA GTT TTG ACA AAA ACC CTA ACA TTC TCC GCC    529
   Met Gly Leu Gly Ala Ser Val Leu Thr Lys Thr Leu Thr Phe Ser Ala
               160                 165                 170
   GCG TTT TCC GCT TTC GGT GAC CCA ATT CCA TGG TTG ATC GCT CTC GCC    577
   Ala Phe Ser Ala Phe Gly Asp Pro Ile Pro Trp Leu Ile Ala Leu Ala
           175                 180                 185
   TTC TTC TTC GCT CGT GGG TTC ATC AAA ACT GGA CTC GGG AAT CGA ATT    625
   Phe Phe Phe Ala Arg Gly Phe Ile Lys Thr Gly Leu Gly Asn Arg Ile
       190                 195                 200
   GCG TAC CAA TTT GTG AAA CTA TTT GGG AGT TCT TCA TTA GGG TTA GGT    673
   Ala Tyr Gln Phe Val Lys Leu Phe Gly Ser Ser Ser Leu Gly Leu Gly
   205                 210                 215                 220
   TAC AGT CTT GTT TTC AGT GAA GCC CTT TTA GCC CCT GCA ATT CCT TCT       721
   Tyr Ser Leu Val Phe Ser Glu Ala Leu Leu Ala Pro Ala Ile Pro Ser
                   225                 230                 235
   GTT TCT GCA AGA GCA GGT GGG ATT TTC CTT CCA TTG GTG AAA TCC CTG       769
   Val Ser Ala Arg Ala Gly Gly Ile Phe Leu Pro Leu Val Lys Ser Leu
               240                 245                 250
   TGT ATT GCT TGT GGT AGC AAT GTG GGT GAT GGA ACT GAG AGG AAA TTG       817
   Cys Ile Ala Cys Gly Ser Asn Val Gly Asp Gly Thr Glu Arg Lys Leu
           255                 260                 265
   GGG GCT TGG TTG ATG CTT ACA TGT TTC CAA ACT TCT GTG ATT TCT AGC       865
   Gly Ala Trp Leu Met Leu Thr Cys Phe Gln Thr Ser Val Ile Ser Ser
       270                 275                 280
   TCT ATG TTT TTA ACT GCT ATG GCT GCT AAT CCT TTG AGT GCA ACT TTG       913
   Ser Met Phe Leu Thr Ala Met Ala Ala Asn Pro Leu Ser Ala Thr Leu
   285                 290                 295                 300
   ACA TTC AAT ACT ATT GGT AAG GCA ATT GGG TGG ATG GAT TGG GCT AAA       961
   Thr Phe Asn Thr Ile Gly Lys Ala Ile Gly Trp Met Asp Trp Ala Lys
                   305                 310                 315
   GCT GCT TTT GTT CCT GGG TTG GTT TCT TTG ATT GTT GTT CCT TTA CTG      1009
   Ala Ala Phe Val Pro Gly Leu Val Ser Leu Ile Val Val Pro Leu Leu
               320                 325                 330
   TTG TAT GTG GTT TAC CCT CCT GAG ATT AAG TCT AGT CCT GAT GCT CCT      1057
   Leu Tyr Val Val Tyr Pro Pro Glu Ile Lys Ser Ser Pro Asp Ala Pro
           335                 340                 345
   CGC CTT GCC AAA GAG AAG TTG GAC AAG ATG GGT CCT ATG ACA AAG AAT      1105
   Arg Leu Ala Lys Glu Lys Leu Asp Lys Met Gly Pro Met Thr Lys Asn
       350                 355                 360
   GAG AGC ATT ATG GCT GTC ACT TTG CTT CTC ACG GTG GGA TTG TGG GTT      1153
   Glu Ser Ile Met Ala Val Thr Leu Leu Leu Thr Val Gly Leu Trp Val
   365                 370                 375                 380
   TTC GGA GGA AAG TTG GGC GTT GAT GCA GTC ACT GCG GCA ATT CTT GGA      1201
   Phe Gly Gly Lys Leu Gly Val Asp Ala Val Thr Ala Ala Ile Leu Gly
                   385                 390                 395
   TTA TCC GTG CTC CTC ATA ACT GGT GTT GTC ACC TGG AAA GAG TGC TTA      1249
   Leu Ser Val Leu Leu Ile Thr Gly Val Val Thr Trp Lys Glu Cys Leu
               400                 405                 410
   GCC GAG TCA GTT GCA TGG GAT ACC CTC ACA TGG TTT GCT GCA CTC ATT      1297
   Ala Glu Ser Val Ala Trp Asp Thr Leu Thr Trp Phe Ala Ala Leu Ile
           415                 420                 425
   GCC ATG GCT GGT TAT CTC AAC AAG TAC GGT CTC ATT ACC TGG TTC AGT      1345
   Ala Met Ala Gly Tyr Leu Asn Lys Tyr Gly Leu Ile Thr Trp Phe Ser
       430                 435                 440
   GAA AAT GTG GTT AAG GTT GTT GGT GGT TTA GGT CTT TCA TGG CAG ATG     1393
   Glu Asn Val Val Lys Val Val Gly Gly Leu Gly Leu Ser Trp Gln Met
   445                 450                 455                 460
   TCA TTC GGC GTT CTA GTT CTT CTT TAC TTC TAC TCT CAC TAC TTC TTT     1441
   Ser Phe Gly Val Leu Val Leu Leu Tyr Phe Tyr Ser His Tyr Phe Phe
                   465                 470                 475
   GCT AGT GGA GCT GCA CAT ATT GGT GCC ATG TTC ACA GCC TTC CTT TCA     1489
   Ala Ser Gly Ala Ala His Ile Gly Ala Met Phe Thr Ala Phe Leu Ser
               480                 485                 490
   GTT GCA AGT GCA CTA GGG ACT CCA CCA TTC TTG GCA GCC ATT GTT CTT     1537
   Val Ala Ser Ala Leu Gly Thr Pro Pro Phe Leu Ala Ala Ile Val Leu
           495                 500                 505
   TCA TTC CTC TCC AAT CTT ATG GGT GGA CTC ACC CAC TAC GGT ATT GGT     1585
   Ser Phe Leu Ser Asn Leu Met Gly Gly Leu Thr His Tyr Gly Ile Gly
       510                 515                 520
   TCA GCC CCC GTT TTC TAC GGT GCT AAC TAT GTT CCC CTA CCA CAA TGG     1633
   Ser Ala Pro Val Phe Tyr Gly Ala Asn Tyr Val Pro Leu Pro Gln Trp
   525                 530                 535                 540
   TGG GGT TAC GGT TTC CTC ATC TCA ATA GTT AAC TTG ATA ATC TGG CTC     1681
   Trp Gly Tyr Gly Phe Leu Ile Ser Ile Val Asn Leu Ile Ile Trp Leu
                   545                 550                 555
   GGA GTT GGG GGA CTT TGG TGG AAA GCT ATT GGA TTG TGG TGATCAGGTT      1730
   Gly Val Gly Gly Leu Trp Trp Lys Ala Ile Gly Leu Trp
               560                 565
   GGTGCTCGTT CGTAGAATTT GTTGACCCTG CAATTTAGTA CTTCTCGCGA ATACTTGCCT   1790
   ATTATATACA AGCTCTTCCT AGAGGTAGCC CTCTGCATTT TTGTTTTTGA TTCTTTGTTT   1850
   CTTAGAAAAT TTAATCCCCC TTTGTCTCTG CGAAATGAAA CAGATCATGA AATATAAAGA   1910
   GCAAAATAGT TCAAATTTGT TTTAAAAAAA AAAAA                              1945
(2) ANGABEN ZU SEQ ID NO: 2:
   (i) SEQUENZKENNZEICHEN:
      (A) LÄNGE: 569 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 2:
   Met Ala Ser Met Ala Leu Ser Leu Thr Ser Ser Pro Thr Tyr Ser Leu
    1               5                  10                  15
   Ser Phe Arg Ser Leu Pro Ser Leu Lys Pro Leu Ser Lys Ser Gln Pro
               20                  25                  30
   Ser Ile Ser Leu Pro Ser Leu Arg Ser Asn Ala Ser Lys Ser Pro Ser
   35 40 45
   Leu Ser His Lys His Phe Leu Ser Pro Pro Ser Leu Leu Leu Pro His
   50 55 60
   Lys Leu Lys Pro Ile Ser Ala Ser Ser Pro Thr Asn Pro Pro Pro Pro
   65 70 75 80
   Pro Ala Pro Val Pro Ser Pro Ala Pro Val Ser Ala Pro Ala Gln Val
   85 90 95
   Gln Pro Trp Gln Gly Ala Ser Ile Lys Pro Leu Leu Ala Ser Ile Leu
   100 105 110
   Thr Gly Val Ile Ile Trp Phe Ile Pro Thr Pro Glu Gly Val Ser Arg
   115 120 125
   Asn Ala Trp Gln Leu Leu Ala Ile Phe Leu Ser Thr Ile Val Gly Ile
   130 135 140
   Ile Thr Gln Pro Leu Pro Leu Gly Ala Val Ala Leu Met Gly Leu Gly
   145 150 155 160
   Ala Ser Val Leu Thr Lys Thr Leu Thr Phe Ser Ala Ala Phe Ser Ala
   165 170 175
   Phe Gly Asp Pro Ile Pro Trp Leu Ile Ala Leu Ala Phe Phe Phe Ala
   180 185 190
   Arg Gly Phe Ile Lys Thr Gly Leu Gly Asn Arg Ile Ala Tyr Gln Phe
   195 200 205
   Val Lys Leu Phe Gly Ser Ser Ser Leu Gly Leu Gly Tyr Ser Leu Val
   210 215 220
   Phe Ser Glu Ala Leu Leu Ala Pro Ala Ile Pro Ser Val Ser Ala Arg
   225 230 235 240
   Ala Gly Gly Ile Phe Leu Pro Leu Val Lys Ser Leu Cys Ile Ala Cys
   245 250 255
   Gly Ser Asn Val Gly Asp Gly Thr Glu Arg Lys Leu Gly Ala Trp Leu
   260 265 270
   Met Leu Thr Cys Phe Gln Thr Ser Val Ile Ser Ser Ser Met Phe Leu
   275 280 285
   Thr Ala Met Ala Ala Asn Pro Leu Ser Ala Thr Leu Thr Phe Asn Thr
   290 295 300
   Ile Gly Lys Ala Ile Gly Trp Met Asp Trp Ala Lys Ala Ala Phe Val
   305 310 315 320
   Pro Gly Leu Val Ser Leu Ile Val Val Pro Leu Leu Leu Tyr Val Val
   325 330 335
   Tyr Pro Pro Glu Ile Lys Ser Ser Pro Asp Ala Pro Arg Leu Ala Lys
               340                 345                 350
   Glu Lys Leu Asp Lys Met Gly Pro Met Thr Lys Asn Glu Ser Ile Met
           355                 360                 365
   Ala Val Thr Leu Leu Leu Thr Val Gly Leu Trp Val Phe Gly Gly Lys
       370                 375                 380
   Leu Gly Val Asp Ala Val Thr Ala Ala Ile Leu Gly Leu Ser Val Leu
   385                 390                 395                     400
   Leu Ile Thr Gly Val Val Thr Trp Lys Glu Cys Leu Ala Glu Ser Val
                   405                 410                 415
   Ala Trp Asp Thr Leu Thr Trp Phe Ala Ala Leu Ile Ala Met Ala Gly
               420                 425                 430
   Tyr Leu Asn Lys Tyr Gly Leu Ile Thr Trp Phe Ser Glu Asn Val Val
           435                 440                 445
   Lys Val Val Gly Gly Leu Gly Leu Ser Trp Gln Met Ser Phe Gly Val
       450                 455                 460
   Leu Val Leu Leu Tyr Phe Tyr Ser His Tyr Phe Phe Ala Ser Gly Ala
   465                 470                 475                     480
   Ala His Ile Gly Ala Met Phe Thr Ala Phe Leu Ser Val Ala Ser Ala
                   485                 490                 495
   Leu Gly Thr Pro Pro Phe Leu Ala Ala Ile Val Leu Ser Phe Leu Ser
               500                 505                 510
   Asn Leu Met Gly Gly Leu Thr His Tyr Gly Ile Gly Ser Ala Pro Val
           515                 520                 525
   Phe Tyr Gly Ala Asn Tyr Val Pro Leu Pro Gln Trp Trp Gly Tyr Gly
       530                 535                 540
   Phe Leu Ile Ser Ile Val Asn Leu Ile Ile Trp Leu Gly Val Gly Gly
   545                 550                 555                     560
   Leu Trp Trp Lys Ala Ile Gly Leu Trp
                   565